# EUROPEAN PATENT APPLICATION

(11) **EP 4 567 098 A1**
(43) Date of publication of application: **11.06.2025**
(21) Application number: 23849812.5
(22) Date of filing: 30.06.2023
(51) Int. Cl.: C12N 1/19, C12N 1/21, C12N 15/90

(54) **HOMOLOGOUS RECOMBINATION METHOD**

(30) Priority: 04.08.2022 JP 2022124956
(71) Applicant: Kabushiki Kaisha Toyota Chuo Kenkyusho, Nagakute-shi, Aichi 480-1192 (JP); TOYOTA JIDOSHA KABUSHIKI KAISHA, Toyota-shi Aichi 471-8571 (JP)
(72) Inventor: ONISHI, Toru, Toyota-shi, Aichi 471-8571 (JP); YASUTANI, Noriko, Toyota-shi, Aichi 471-8571 (JP)
(74) Representative: J A Kemp LLP
(86) International application number: PCT/JP2023/024424
(87) International publication number: WO 2024/029253

(57) **Abstract**

There is provided a homologous recombination method of excising a specific DNA sequence from a DNA molecule, comprising: providing the DNA molecule that has a pair of target sequences as a target of a site-specific recombination enzyme are placed in different directions from each other across the specific DNA sequence and that a pair of homologous sequences as DNA sequences sharing homology with each other are placed on further outside of the pair of target sequences placed across the specific DNA sequence; and making the site-specific recombination enzyme act on the DNA molecule and thereby causing homologous recombination between the pair of homologous sequences, so as to excise the specific DNA sequence along with the pair of target sequences from the DNA molecule.

## Description

### Technical Field

The present disclosure relates to a homologous recombination method.

### Background Art

A variety of techniques have been conventionally known in relation to recombination in a DNA molecule. For example, it is known that a homologous recombination method using recombination occurring between base sequences having a high degree of homology is applicable to a relatively wide range of organism species from prokaryotes to animals (as shown in, for example, Non-Patent Literatures 1 to 4). In another example, site-specific recombination using a site-specific recombination enzyme is known as a method of excising a specific DNA sequence from a DNA molecule (as shown in, for example, Non-Patent Literatures 5 and 6). Furthermore, another known method of excising a specific DNA sequence places an object sequence to be excised between transposon-specific inverse terminal sequences and uses transposase to excise the object sequence along with the inverse terminal sequences (as shown in, for example, Non-Patent Literature 7).

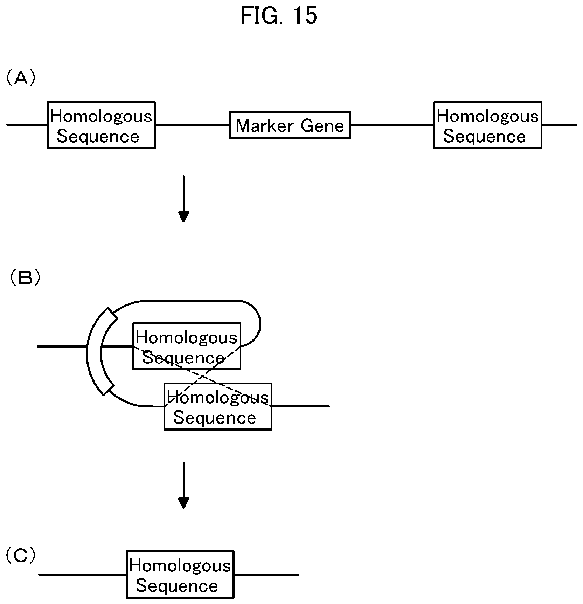

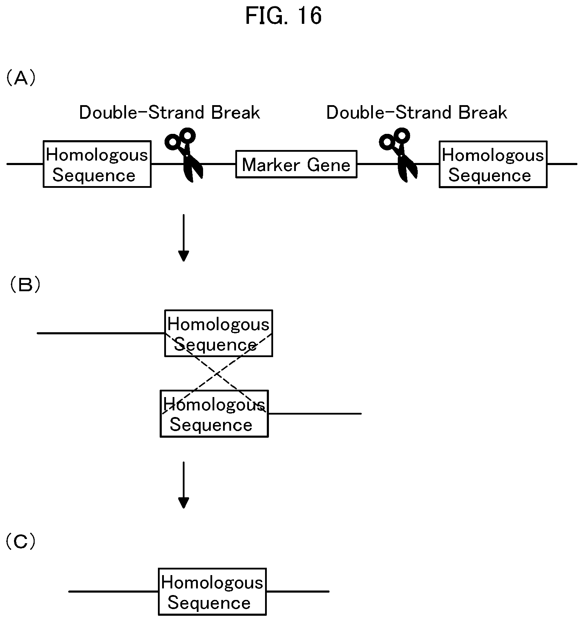

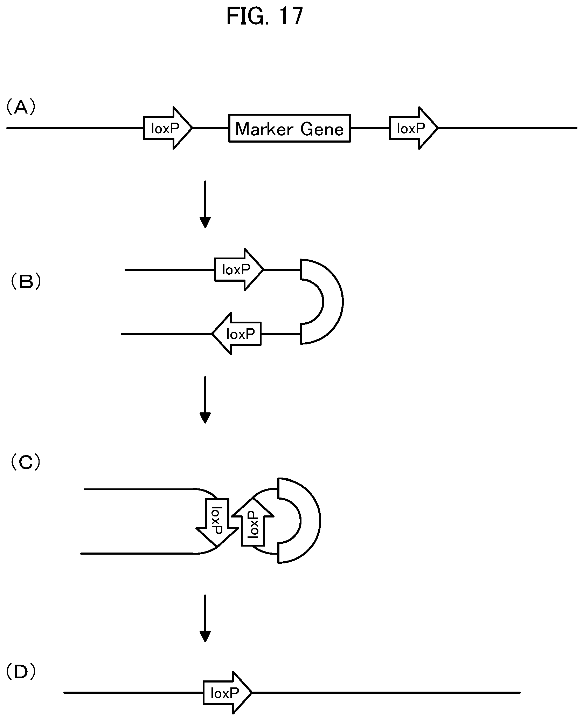

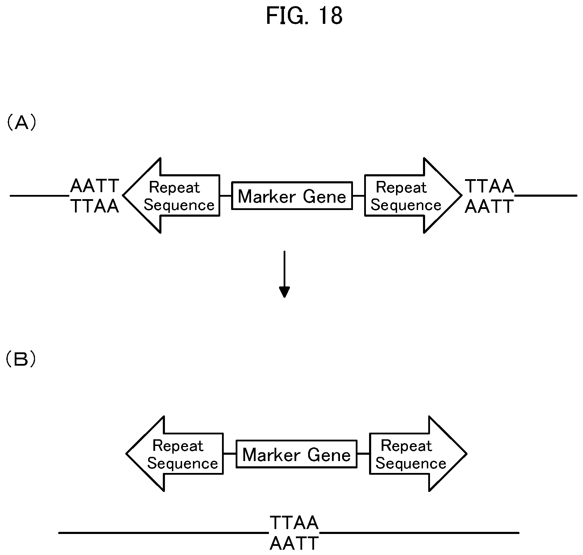

Fig. 15 is an explanatory diagram illustrating the state of performing homologous recombination between a pair of homologous sequences on an identical DNA molecule to incise a marker gene between the homologous sequences, as one example of homologous recombination. Fig. 16 is an explanatory diagram illustrating a homologous recombination method using a genome editing technique, as another example of homologous recombination. This method uses a genome editing technique (for example, ZFN, TALEN, CRISPR-Cas) to incise a DNA in the vicinity of homologous recombination target sequences (homologous sequences). This activates homologous recombination repair and enhances the efficiency of homologous recombination via the target sequences. Fig. 17 is an explanatory diagram illustrating a site-specific recombination reaction. Fig. 17 illustrates a site-specific recombination reaction in a Cre-loxP recombination system using Cre recombinase and loxP sequences as an example. This site-specific recombination reaction enables a DNA sequence between target sequences such as loxP sequences (a DNA sequence including a marker gene in the illustrated example of Fig. 17) to be incised at such a high efficiency that does not require, for example, screening by the marker gene. Fig. 18 is an explanatory diagram illustrating an example of a reaction using the excision enzyme of transposon. Fig. 18 illustrates an excision reaction by transposon PiggyBac as an example. This reaction allows for excision of a DNA sequence (a DNA sequence including a marker gene in Fig. 18) between specific inverse terminal sequences (repetition sequences) that are recognizable by an excision enzyme (transposase). This reaction enables this DNA sequence to be excised at a high efficiently without leaving a trail sequence after the reaction, unlike the site-specific recombination reaction shown in Fig. 17.

### Citation List

### Non-Patent Literatures

Non-Patent Literature 1: Francesca Storici et al., Proc. Natl. Acad. Sci. USA, 100, 14994 - 14999 (2003)
Non-Patent Literature 2: Justin M. Vento et al., J. Ind. Microbiol. Biot., 46, 1327 - 1341 (2019)
Non-Patent Literature 3: Dana Carroll, Annual REview of Biochemistry, 83, 409 - 439 (2014)
Non-Patent Literature 4: Hiroshi Ochiai et al., Experimental Medicine (Jikken Igaku), 31, 95 - 100 (2013)
Non-Patent Literature 5: Yueju Wang et al., Plant Cell Rep., 30, 267 - 285 (2011)
Non-Patent Literature 6: Xueying Tian et al., J. Biol. Chem., 296, 100509 (2021)
Non-Patent Literature 7: Sheng Ding et al., Cell, 122, 473 - 483 (2005)

### Summary

### Technical Problem

For example, the homologous recombination technique that utilizes the homologous recombination repair mechanism without DNA incision as shown in Fig. 15, however, needs limitation of the target to organism species having high homologous recombination repair activity, such as Saccharomyces cerevisiae, chicken DT40 cells, and mouse ES cells. Even Saccharomyces cerevisiae known to have an especially high efficiency have a relatively low recombination frequency of about 10⁻⁶ as described in Non-Patent Literature 1. Screening using a marker gene is accordingly required to obtain a homologous recombination strain. In the homologous recombination method using the genome editing technique shown in Fig. 16, on the other hand, priority is given to the non-homologous terminal linkage over the homologous recombination repair in a lot of organisms, and the incised sites are directly linked with each other to repair. In many cases, these results in the insufficient frequency of a desired recombination reaction and requires screening using the marker gene. Furthermore, the homologous recombination method using the genome editing technique has a problem that a series of operations including, for example, production of vectors and induction of DNA incision, are relatively complicated.

Furthermore, the site-specific recombination reaction using the site-specific recombination enzyme shown in Fig. 17 determines the target sequences according to the site-specific recombination enzyme used. This accordingly has problems that the target sequences cannot be freely designed and that the target sequence remains in a DNA molecule after incision of the DNA sequence by recombination. The remaining target sequence is likely to cause a problem that unintentional recombination reaction occurs in the process of using the DNA molecule after recombination for a similar site-specific recombination reaction.

Moreover, the reaction utilizing the incision enzyme of transposon shown in Fig. 18 has a problem that the location of incision in the DNA sequence is limited to a specific target sequence on the genome (four bases of TTAA in the illustrated example of Fig. 18). There has accordingly been a demand for a recombination technique that allows for recombination at the higher efficiency, that has no limitation on a target DNA sequence, that does not cause a trail sequence to remain in a molecule after incision of a DNA sequence, and that is performed by simpler operations.

### Solution to Problem

The present disclosure may be implemented by the following aspects.
(1) According to one aspect of the present disclosure, there is provided a homologous recombination method of excising a specific DNA sequence from a DNA molecule. This homologous recombination method comprises providing the DNA molecule that has a pair of target sequences as a target of a site-specific recombination enzyme are placed in different directions from each other across the specific DNA sequence and that a pair of homologous sequences as DNA sequences sharing homology with each other are placed on further outside of the pair of target sequences placed across the specific DNA sequence; and making the site-specific recombination enzyme act on the DNA molecule and thereby causing homologous recombination between the pair of homologous sequences, so as to excise the specific DNA sequence along with the pair of target sequences from the DNA molecule.
   The homologous recombination method of this aspect takes advantage of a site-specific recombination reaction, which uses a site-specific recombination enzyme having a relatively high efficiency of recombination, to increase the frequency of homologous recombination, i.e., the efficiency of recombination. This recombination reaction causes homologous recombination and accordingly suppresses restriction on a DNA sequence as a target of the recombination reaction. Furthermore, the homologous recombination method of this aspect excises the target sequences that are placed closer to the specific DNA sequence than the homologous sequences, accompanied with excision of the specific DNA sequence. This configuration suppresses a trail sequence from remaining in a molecule after excision of the DNA sequence. Moreover, the characteristic sequence structure including the specific DNA sequence, the target sequences and the homologous sequences as described above can be obtained by a relatively simple process using the general gene recombination technology.
(2) In the homologous recombination method of the above aspect, the site-specific recombination enzyme may be an enzyme belonging to an integrase family and may cause a reversible reaction to proceed as a recombination reaction of the target sequences. This configuration improves the effect of increasing the frequency of homologous recombination.
(3) In the homologous recombination method of the above aspect, the site-specific recombination enzyme may be an enzyme belonging to a bidirectional tyrosine recombinase subfamily. This configuration improves the effect of increasing the frequency of homologous recombination.
(4) In the homologous recombination method of the above aspect, the site-specific recombination enzyme may be an enzyme selected among Cre recombinase, FLP recombinase, R recombinase and Dre recombinase. This configuration improves the effect of increasing the frequency of homologous recombination.
(5) The homologous recombination method of the above aspect may further comprise using Saccharomyces cerevisiae or Escherichia coli as a target cell of homologous recombination, wherein a distance between the target sequence and the homologous sequence placed adjacent to each other either on an upstream side or on a downstream side of the specific DNA sequence may be 1000 bp or less. This configuration enables the effect of enhancing the efficiency of recombination in the above layout of the specific DNA sequence, the pair of target sequences in the different directions from each other, and the pair of homologous sequences to be ensured by adjusting the distance described above.
(6) In the homologous recombination method of the above aspect, the homologous sequence used may be a sequence having a length of 60 bp or less. This configuration enhances the efficiency of homologous recombination even in the case of using short homologous sequences, which comparatively have a difficulty in performing homologous recombination.
(7) In the homologous recombination method of the above aspect, the homologous sequence used may be a sequence having a length of 25 bp or greater. This configuration enables the effect of enhancing the efficiency of recombination in the above layout of the specific DNA sequence, the pair of target sequences in the different directions from each other, and the pair of homologous sequences to be ensured by adjusting the length of the homologous sequences described above.

The present disclosure may be implemented by a variety of aspects other than those described above, for example, by an aspect of a vector used for homologous recombination or an aspect of a recombinant obtained by homologous recombination.

### Brief Description of Drawings

Fig. 1 is an explanatory diagram schematically illustrating a DNA sequence structure in relation to a homologous recombination method according to an embodiment;
Fig. 2 is an explanatory diagram illustrating one example of homologous recombination performed by the homologous recombination method according to the embodiment;
Fig. 3 is an explanatory diagram illustrating a destination vector for level 0;
Fig. 4 is an explanatory diagram illustrating destination vectors for level 1;
Fig. 5 is an explanatory diagram illustrating a destination vector for level 2;
Fig. 6 is an explanatory diagram illustrating the state of introducing the sequence structure of Fig. 1 into the Saccharomyces genome;
Fig. 7 is an explanatory diagram illustrating the state of introducing a sequence structure of a comparative example into the Saccharomyces genome;
Fig. 8 is an explanatory diagram illustrating the state of introducing the sequence structure of Fig. 1 into the Saccharomyces genome;
Fig. 9 is an explanatory diagram showing the configurations of respective level 1 modules used to be introduced into the Saccharomyces genome;
Fig. 10 is an explanatory diagram showing the configurations of V3P vector sets introduced into the Saccharomyces genome;
Fig. 11 is an explanatory diagram illustrating a level 2 vector for Escherichia coli used for the purpose of evaluation of the efficiency of homologous recombination;
Fig. 12 is an explanatory diagram showing the details of level 2 vectors introduced into Escherichia coli;
Fig. 13 is an explanatory diagram showing the results of an experiment of homologous recombination in Saccharomyces cerevisiae;
Fig. 14 is an explanatory diagram showing the results of an experiment of homologous recombination in Escherichia coli;
Fig. 15 is an explanatory diagram illustrating the state of homologous recombination between homologous sequences;
Fig. 16 is an explanatory diagram illustrating a homologous recombination method utilizing a genome editing technique;
Fig. 17 is an explanatory diagram illustrating a site-specific recombination reaction; and
Fig. 18 is an explanatory diagram illustrating a reaction utilizing an excision enzyme of transposon.

### Description of Embodiments

### A. First Embodiment

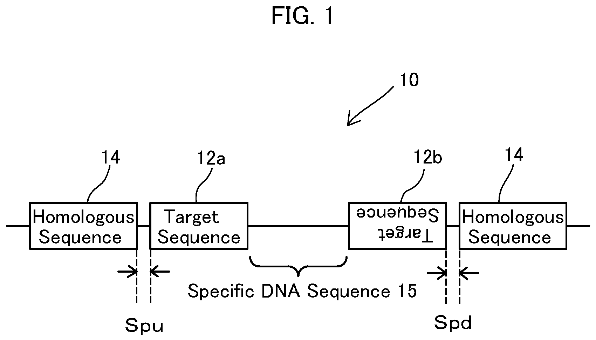

Fig. 1 is an explanatory diagram schematically illustrating a DNA sequence structure in relation to a homologous recombination method according to an embodiment. The homologous recombination method of the embodiment is a method of combining site-specific recombination with homologous recombination to excise a specific DNA sequence from a DNA molecule. As shown in Fig. 1, according to the embodiment, in a DNA molecule 10 that is an object of homologous recombination, one pair of target sequences 12a and 12b that are the target of a site-specific recombination enzyme (sequence-specific recombination enzyme) are placed in different directions from each other across a specific DNA sequence 15 that is an object to be excised. One pair of homologous sequences 14 that are DNA sequences sharing homology with each other are placed on further outside of the pair of target sequences 12a and 12b that are placed in reverse directions across the specific DNA sequence 15. The homologous recombination method of the embodiment makes the site-specific recombination enzyme described above act on this DNA molecule 10 and thereby causes homologous recombination to proceed between the pair of homologous sequences 14, so as to excise the specific DNA sequence 15, along with the pair of target sequences 12a and 12b, from the DNA molecule 10.

The target sequences 12a and 12b may be appropriately set according to the site-specific recombination enzyme (site-specific recombinase) used. The site-specific recombination enzyme used herein is desirably a recombination enzyme that causes a reversible reaction to proceed as a recombination reaction proceeding between the pair of target sequences 12a and 12b. The state that "a reversible reaction proceeds" herein means that another recombination reaction for returning to the state prior to an original recombination reaction is allowed to proceed after the original recombination reaction.

For example, a recombinase belonging to an integrase family may be used as the site-specific recombination enzyme that causes the "reversible reaction to proceed". Site-specific recombinases belonging to the integrase family is classified into two groups: a tyrosine recombinase family and a serine recombinase family (as shown in Non-Patent Literature 5). These site-specific recombinases are classified, based on whether an active site in a catalyst domain of the enzyme has a tyrosine residue or has a serine residue. The tyrosine recombinase family is further classified based on the action mechanism thereof, into a bidirectional tyrosine recombinase subfamily (hereinafter also referred to as "bidirectional tyrosine subfamily") and a unidirectional tyrosine recombinase subfamily (hereinafter also referred to as "unidirectional tyrosine subfamily"). The serine recombinase family is further classified based on the size, into a large serine recombinase subfamily (hereinafter also referred to as "large serine subfamily") and a small serine recombinase family (hereinafter also referred to as "small serine subfamily").

Examples of the combination of the bidirectional tyrosine subfamily and the target sequence include a Cre-loxP recombinant system using the Cre recombinase and a loxP sequence; an FLP-FRT recombinant system using the FLP recombinase and an FRT sequence; an R-RS recombinant system using the R recombinase and an RS sequence; and a Dre-rox recombinant system using the Dre recombinase and a rox sequence. Genetic crossover via such a bidirectional tyrosine subfamily occurs between two identical target sequences (recognition sites). The recombination reaction is thus fully reversible, because of the identical recognition sites.

Examples of the unidirectional tyrosine subfamily include λ recombinase, HK101 recombinase and pSAM2 recombinase. Examples of the large serine subfamily include PhiC31 recombinase, TP901-1 recombinase, Bxb1 recombinase and R4 recombinase. Such a unidirectional tyrosine subfamily or large serine subfamily acts on two recognition sites of different sequences known as attB and attP and generates hybrid recombination sites known as attL and attR (ACS Synth. Biol. 7, 299-310 (2018)). In this manner, the sequences attB and attP of the recognition sites are changed to the sequences attL and attR, so that only the recombinase does not cause a reverse reaction. Addition of an RDF (recombination directionality factor), however, changes over the directionality of recombination and allows for a reversible reaction. In this state, regulating the concentrations of the recombinase and the RDF allows for adjustment of the reaction rates in the respective reactions (Nucleic Acid Res. 44(15), 7360-7372 (2016)) and enables a reversible reaction to proceed continuously.

As described above, the recombinase belonging to the integrase family may be preferably used as the site-specific recombination enzyme that continues the reversible reaction. More specifically, using the bidirectional tyrosine subfamily can more readily continue the reversible reaction by a simple reaction system without using any additional constituent element, such as the RDF. The small serine subfamily described above has only one directionality of the reaction and has no known method for reversing the reaction. This embodiment accordingly does not use the small serine subfamily to make a reversible reaction proceed.

The degree of homology between the pair of homologous sequences 14 is not specifically limited, as long as the degree of homology allows for homologous recombination. The degree of homology that allows for homologous recombination is affected by, for example, the length of the homologous sequence. The degree of homology is, for example, at least 80% or higher, is preferably 85% or higher, is more preferably 90% or higher, is furthermore preferably 95% or higher and is most preferably equal to 100%. The length of the homologous sequence 14 is not specifically limited, as long as the length of the homologous sequence allows for homologous recombination. In terms of suppressing the occurrence of any unintentional recombination reaction, the length of the homologous sequence 14 is, for example, 20 bp or greater, is preferably 30 bp or greater and is more preferably 40 bp or greater. In terms of enhancing the efficiency of homologous recombination between the homologous sequences 14, the length of the homologous sequence 14 is preferably 25 bp or greater and is more preferably 30 bp or greater. In terms of facilitating production of a vector or the like having a desired sequence structure, the length of the homologous sequence 14 is, for example, 500 bp or less, is preferably 400 bp or less and is more preferably 300 bp or less. In general, the excessively short homologous sequence 14 suppresses the homologous recombination from proceeding and lowers the efficiency of recombination. The homologous recombination method of the embodiment, however, achieves the high efficiency of homologous recombination even when the length of the homologous sequence 14 is, for example, 60 bp or less or is even 50 bp or less.

As shown in Fig. 1, a first spacer sequence Spu may be provided between a 5'-terminal of the target sequence 12a, which is placed on an upstream side (on a 5'-side) of the specific DNA sequence 15 out of the target sequences 12a and 12b, and a 3'-terminal of the homologous sequence 14 close to the target sequence 12a out of the pair of homologous sequences 14. A second spacer sequence Spd may be provided between a 3'-terminal of the target sequence 12b, which is placed on a downstream side (on a 3'-side) of the specific DNA sequence 15 out of the target sequences 12a and 12b and a 5'-terminal of the homologous sequence 14 close to the target sequence 12b out of the pair of homologous sequences 14. The length of the first spacer sequence Spu and the length of the second spacer Spd, i.e., the distance between the target sequence and the homologous sequence placed next to each other on either the upstream side or on the downstream side of the specific DNA sequence 15, is 0 bp or greater. This distance may be, for example, 50 bp or greater and may also be 1500 bp or less.

For example, in the case where homologous recombination is performed in Saccharomyces cerevisiae (budding yeast) or in Escherichia coli by using the Cre recombinase and the loxP sequence as the combination of the recombinase and the target sequence, the distance described above may be, for example, 1500 bp or less, may further be 1200 bp or less and is preferably 1000 bp or less. The lengths of the first spacer sequence Spu and the second spacer sequence Spd described above are set appropriately according to the site-specific recombination enzyme and the target sequence thereof, the type of the cell involved in the recombination, the gene locus involved in the recombination in the cell, and the like. This enhances the efficiency of recombination by homologous recombination.

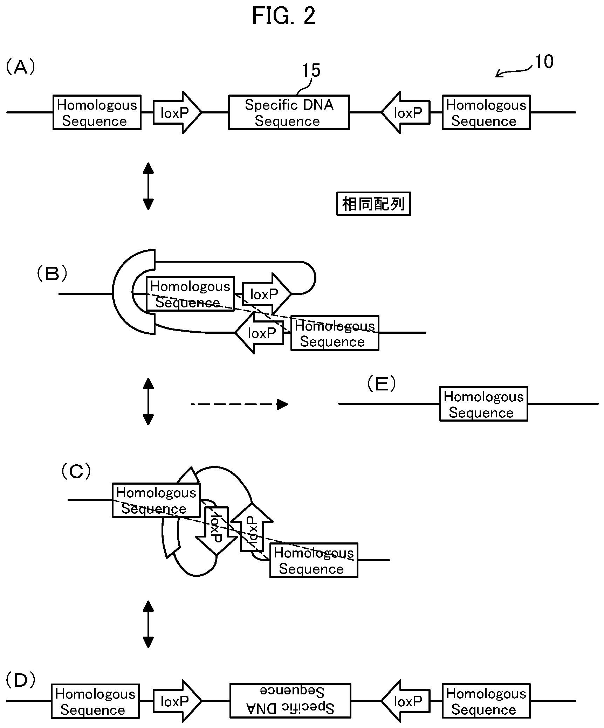

Fig. 2 is an explanatory diagram illustrating one example of the homologous recombination that occurs by the homologous recombination method of the embodiment and that uses the Cre recombinase and the loxP sequence as the combination of the recombinase and the target sequence. The action of a site-specific recombination enzyme (the Cre recombinase in the illustrated example of Fig. 2) in the state of Fig. 2(A) that is equivalent to the state of Fig. 1 causes homologous recombination between homologous sequences and excises a specific DNA sequence 15 along with target sequences (loxP sequences) from a DNA molecule 10 (as shown in Fig. 2(E)). The mechanism that causes such homologous recombination to proceed is thought to be as follows.

When the Cre recombinase acts as described above, a reversible recombination reaction proceeds between a pair of target sequences (loxP sequences in the illustrated example of Fig. 2) in one identical DNA molecule 10 that has a relatively close distance between the target sequences, and inversion of a specific DNA sequence 15 continues. The destabilization of DNA caused by continuation of the reversible recombination reaction between the target sequences as described above is likely to activate a DNA repair mechanism by homologous recombination and is thought to induce homologous recombination between a pair of homologous sequences placed close to a pair of target sequences in a DNA molecule 10. Furthermore, repeating the closeness in the distance between the homologous sequences is also likely to contribute to increasing the frequency of homologous recombination. This increases the frequency of homologous recombination in the process of continuation of the reversible recombination reaction between the target sequences. In other words, homologous recombination is thought to proceed at a significantly high frequency than the frequency of natural homologous recombination in the state that the reversible reaction between the target sequences does not proceed. Fig. 2 illustrates an example of repeating the reaction of inverting the "specific DNA sequence 15" between the loxP sequences placed in reverse directions by a change from the state of Fig. 2(A) to the state of Fig. 2(D). In the case where a pair of loxP sequences are placed in an identical direction unlike the example of Fig. 2(A), a reaction proceeds for excision of the specific DNA sequence 15 by the site-specific recombination between the pair of loxP sequences. This excision reaction proceeds at an extremely high frequency, compared with a reverse reaction of combining the excised specific DNA sequence 15 at the position prior to the excision in the DNA molecule 10. Accordingly, most DNA molecules 10 are stabilized in the state that the specific DNA sequence 15 is excised between pair of target sequences (loxP sequences). In the case where the pair of loxP sequences are placed in reverse directions like this embodiment, on the other hand, the repetition of the reversal reaction of the specific DNA sequence 15 continues the unstable state of DNA and causes the distance between the pair of homologous sequences 14 to be the close distance for a longer time period. This accordingly improves the efficiency of homologous recombination that excises the specific DNA sequence 15 along with the target sequences placed between the homologous sequences.

For example, when the specific DNA sequence 15 includes a marker gene such as chemical resistance, a cell including the DNA molecule 10 involved in the homologous recombination may be selected by using the expression of this marker gene as an indication. In another example, when the specific DNA sequence includes a gene of a site-specific recombination enzyme, a site-specific recombination enzyme is generated and the site-specific recombination reaction continues, in the state that the gene of the site-specific recombination enzyme is allowed to express, while the reversible recombination reaction is continuously performed between the target sequences. After the homologous recombination, the specific DNA sequence 15 including the gene of the site-specific recombination enzyme is excised. This suppresses the effect of the gene of the above enzyme that becomes unnecessary.

The homologous recombination method of the embodiment configured as described above uses the DNA molecule 10 where the pair of target sequences 12a and 12b are placed in reverse directions across the specific DNA sequence 15 and that the pair of homologous sequences 14 are placed on the further outside of the pair of target sequences 12a and 12b placed across the specific DNA sequence 15. The homologous recombination method of the embodiment makes the site-specific recombination enzyme act on this DNA molecule 10 and thereby causes homologous recombination between the pair of homologous sequences 14, so as to excise the specific DNA sequence 15 from the DNA molecule 10. In general, the site-specific recombination proceeding between the target sequences 12a and 12b has a significantly higher frequency of the recombination reaction, compared with the homologous recombination proceeding between the homologous sequences 14. As shown in Fig. 2(B) and Fig. 2(D), the distance between the pair of homologous sequences 14 placed on the outside of the target sequences 12a and 12b in the identical DNA molecule 10 is shortened in the course of the site-specific recombination reaction proceeding between the target sequences 12a and 12b in the identical molecule. As a result, this increases the frequency of the homologous recombination between the homologous sequences 14 and facilitates excision of the specific DNA sequence 15 from the DNA molecule 10 by the homologous recombination. Especially, using an enzyme that belongs to the integrase family and that causes a reversible reaction to proceed as the recombination reaction between the target sequences 12a and 12b, for the site-specific recombination enzyme shortens the distance between the pair of homologous sequences 14 and improves the effect of increasing the frequency of the homologous recombination.

Moreover, the configuration of the embodiment uses the reaction of the site-specific combination enzyme and performs homologous recombination between the pair of homologous sequences 14 placed on the outside of the target sequences 12a and 12b, so as to excise the specific DNA sequence 15. This configuration enables the target sequences 12a and 12b to be excised along with the specific DNA sequence from the DNA molecule 10. Neither of the target sequences 12a and 12b accordingly remain in the DNA molecule 10 after excision of the specific DNA sequence 15. For example, even in the case where a similar site-specific combination technique is applied to the DNA molecule 10 after excision of the specific DNA sequence 15, this configuration suppresses an unintentional recombination reaction from occurring due to the remaining target sequence.

Furthermore, the homologous recombination method of the embodiment implements the excision of the specific DNA sequence 15 by homologous recombination and thereby ensures the degree of freedom in design of the sequence for excision (the homologous sequence 14), unlike the method of excising the specific DNA sequence 15 by site-specific recombination using a site-specific recombination enzyme. The homologous recombination method of the embodiment enables a series of operations for obtaining a sequence structure where genes are stacked as shown in Fig. 1 (for example, a series of operations including production of a vector and induction of DNA breakage) to be performed by a relatively simple process using a general genetic engineering technique. Any of various known methods that allow a plurality of DNA fragments to be connected with one another irrespective of the terminal configuration of DNA, may be employed for the method of stacking genes to obtain the sequence structure shown in Fig. 1. More specifically, a cloning process for obtaining the sequence structure of Fig. 1 may be performed by, for example, a traditional method using a restriction enzyme, the Golden Gate method, or a method using a Gibson Assembly system (Nature Methods 6 (5), 343-345 (2009)), an NEBuilder HiFi DNA Assembly Cloning Kit (manufactured by New England Biolabs), or an In-Fusion HD Cloning Kit (manufactured by Takara Bio Inc.) (In-Fusion is a registered trademark). The configuration of the embodiment accordingly achieves the recombination at the higher efficiency, suppresses the restriction on the DNA sequence as a target of the recombination reaction, suppresses a trail sequence from remaining in a molecule after excision of the DNA sequence, and allows recombination of DNA to be performed by a simpler operation, unlike the conventionally known DNA recombination methods described above with reference to Fig. 15 to Fig. 18.

Furthermore, the homologous recombination method of the embodiment achieves the high efficiency of recombination in systems using a wider range of cells including prokaryotes, eukaryotes, animal cultured cells, as well as in vitro systems. For example, in conventionally known homologous recombination, the efficiency of recombination significantly varies according to the organism species and the cell type. Even Saccharomyces cerevisiae known to have a relatively high efficiency of homologous recombination had a recombination frequency of about 10⁻⁶ as described in Non-Patent Literature 1. In Escherichia coli, a method of performing genetic transformation by introducing and inducing a Lambda phage-derived homologous recombination enzyme is known as the most efficient genome homologous recombination method. Even this method, however, had the efficiency of about 10⁻³ (Front. Microbiol., 11 September 2020, https://doi.org/10.3389/fmicb.2020.548410). The homologous recombination method of the embodiment, however, uses a general site-specific recombination enzyme, which is applicable to a wide range of cell types or the like, so as to achieve homologous recombination at the higher efficiency of, for example, about 10⁻⁰ to 10⁻², without limiting the combination of the cell as the object of recombination and the enzyme to a specific combination as in the case of using the Lambda phage-derived homologous recombination enzyme for Escherichia coli.

As described above, the homologous recombination method of the embodiment uses a variety of site-specific recombination enzymes to perform the homologous recombination between the homologous sequences 14. Especially preferable is a system using the Cre recombinase that is widely used for a variety of organism species including in vivo systems and that is known to show a wide range of reactivity. The system using the Cre recombinase enhances the efficiency of homologous recombination for a wide range of organism species irrespective of the prokaryote or the eukaryote or irrespective of the animal cell or the plant cell. Not only the system using the Cre recombinase, but a system using any one of the FLP recombinase, the R recombinase and the Dre recombinase that belong to the bidirectional tyrosine subfamily is also widely usable for a variety of organism species. Among the recombinases belonging to the bidirectional tyrosine subfamily, for example, each of Dre, VCre, SCre, Vika and Nigri recombinases has a gene that is a homologous gene sharing homology with the gene of the Cre recombinase and is known to have a substantially similar action mechanism to the action mechanism of the Cre recombinase, regardless of different target sequences. It is also desirable to use such a recombinase obtained from a homologous gene with the gene of the Cre recombinase, since using this recombinase also effectively enhances the efficiency of homologous recombination in a wide variety of organism species, as in the case of using the Cre recombinase.

### Examples

The following describes the present disclosure more specifically by referring to some examples. The present disclosure is, however, not limited to the description of these examples. In the examples described below, a vector having the sequence structure shown in Fig. 1 was produced; and the efficiencies of homologous recombination in both a prokaryote and a eukaryote were examined by integrating the sequence structure of the vector into the genome of a Saccharomyces cerevisiae BY4742 strain as a haploid of laboratory yeast or by introducing the sequence structure of the vector as a plasmid into Escherichia coli DH5α strain or BL21 (DE3) strain.

### <Production of Vector Set for Cloning>

Vectors used for the test were all built by the Golden Gate method (PLos ONE 6, e16765 (2011)). The Golden Gate method is a known method of stacking genes that uses Type IIS restriction enzyme and T4 DNA ligase to insert a plurality of DNA fragments into a vector in a sequence designed in advance. Vectors corresponding to a destination vector for level 0, destination vectors for level 1 and a destination vector for level 2 were produced as subcloning vectors required for the Golden Gate method. The following sequentially describes procedures of producing the destination vector for level 0, the destination vectors for level 1 and the destination vector for level 2.

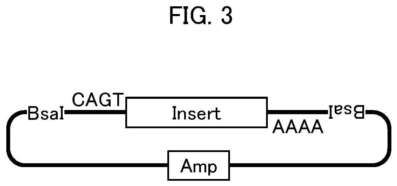

Fig. 3 is an explanatory diagram illustrating a destination vector for level 0. As shown in Fig. 3, the destination vector for level 0 is a vector including a Bsal recognition site. The destination vector for level 0 was produced by amplifying a target DNA fragment by PCR using pUC 19 or a synthetic DNA as a template. A primer used for PCR was artificially synthesized by adding a DNA sequence such as to have an overlap of approximately 15 bp with an adjacent DNA sequence and thereby binding respective DNA fragments with one another. The destination vector for level 0 was completed by sequentially binding the amplified PCR fragments to one another using the In-Fusion HD Cloning Kit (manufactured by Takara Bio Inc.) (In-Fusion is a registered trademark).

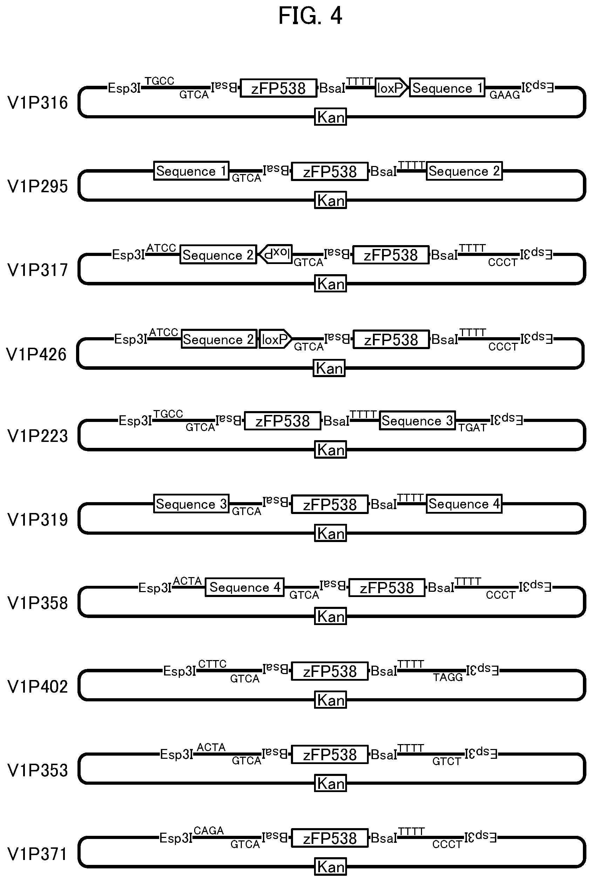

Fig. 4 is an explanatory diagram illustrating destination vectors for level 1. As shown in Fig. 4, the destination vectors for level 1 are a set of multiple different types of vectors. Each of the vectors has a zFP538 gene of a fluorescent protein (Nat Biotechnol. 17, 969-973 (1999)) and BsaI recognition sites placed in reverse directions across this zFP538 gene. The respective vectors included in the destination vector for level 1 have different loxP sequences, different sequences 1 to 4 described below, and differences in the presence or absence, the combination and the layout of a restriction enzyme site used for the Golden Gate reaction and a cleavage site. These destination vectors for level 1 were produced by amplifying target DNA fragments using pUC19 and a synthetic DNA (including a kanamycin-resistant gene) as templates and sequentially binding the DNA fragments by using the In-Fusion HD Cloning Kit. The sequences 1 to 4 described above are homologous sequences of 57 bp that are provided at terminals of respective amplified fragments obtained by PCR amplification in order to bind the respective amplified fragments in a desired sequence. Any arbitrary sequences may be employed for the sequences 1 to 4, as long as these sequences have sufficiently low similarity. Artificial sequences having little similarity to the genome sequence of Saccharomyces cerevisiae were set in this example: sequences described in Microb Cell Fact 12, 47 (2013) were used for the sequence 1 and the sequence 2; and sequences described in Nucleic Acids Res. 43, 6620 - 6630 (2015) were used for the sequence 3 and the sequence 4.

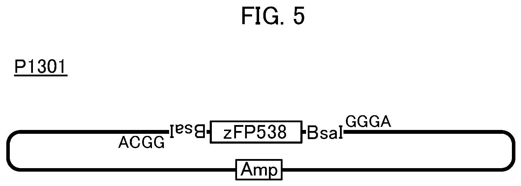

Fig. 5 is an explanatory diagram illustrating a destination vector P1301 for level 2. The destination vector for level 2 is a vector used for an examination in Escherichia coli, which repeats the Golden Gate reaction twice to produce a vector. The destination vector for level 2 was produced by a similar procedure to the destination vector for level 0 and the destination vectors for level 1 described above.

### < Production of Vector Set for Saccharomyces cerevisiae>

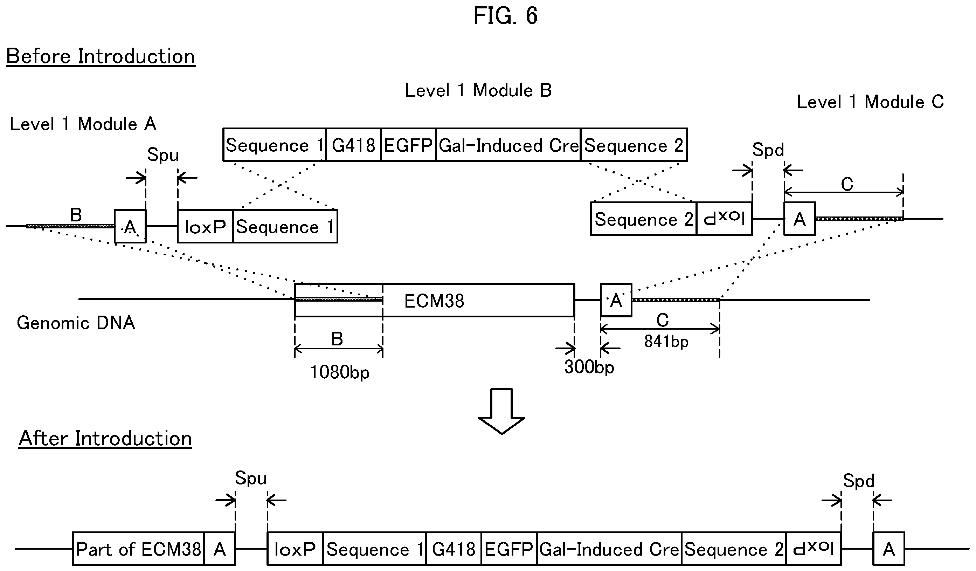

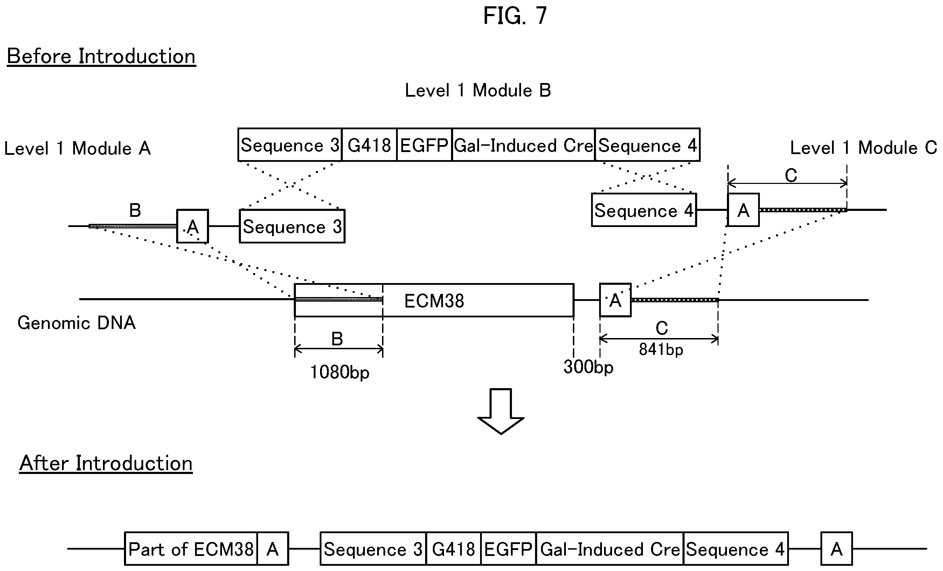

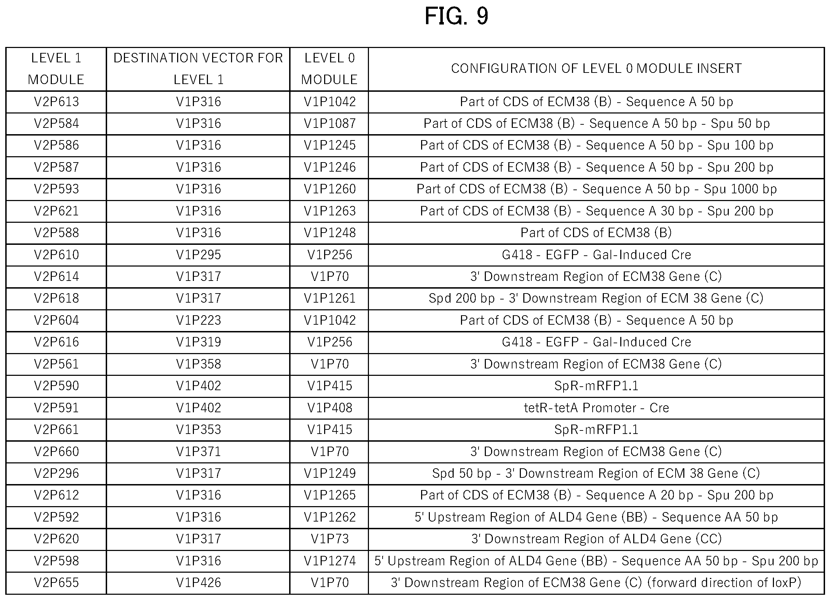

Fig. 6 is an explanatory diagram illustrating the state of introducing the sequence structure shown in Fig. 1 into an ECM38 gene locus of the Saccharomyces genome by homologous recombination of a level 1 module, which is produced by using the destination vector for level 0 and the destination vectors for level 1 described above. Fig. 7 is an explanatory diagram illustrating the state of similarly introducing a sequence structure of a comparative example into the Saccharomyces genome. In this illustrated example, loxP sequences are used as the target sequences 12a and 12b, and the homologous sequence 14 is shown as a homologous sequence A. As shown in Fig. 6 and Fig. 7, the homologous sequence A used was a sequence in a range of 20 bp, in a range of 30 bp or in a range of 50 bp from 301 bp on a 3'-terminal side of an ECM38 protein coding region (CDS) in the Saccharomyces genomic DNA as an object to be introduced. A sequence having the length of 20 bp, a sequence having the length of 30 bp, or a sequence having the length of 50 bp was accordingly used as the homologous sequence A (as shown in Fig. 9, Fig. 10 and Fig. 13 described later). The sequence structure of the comparative example shown in Fig. 7 does not include the loxP sequences as the target sequences.

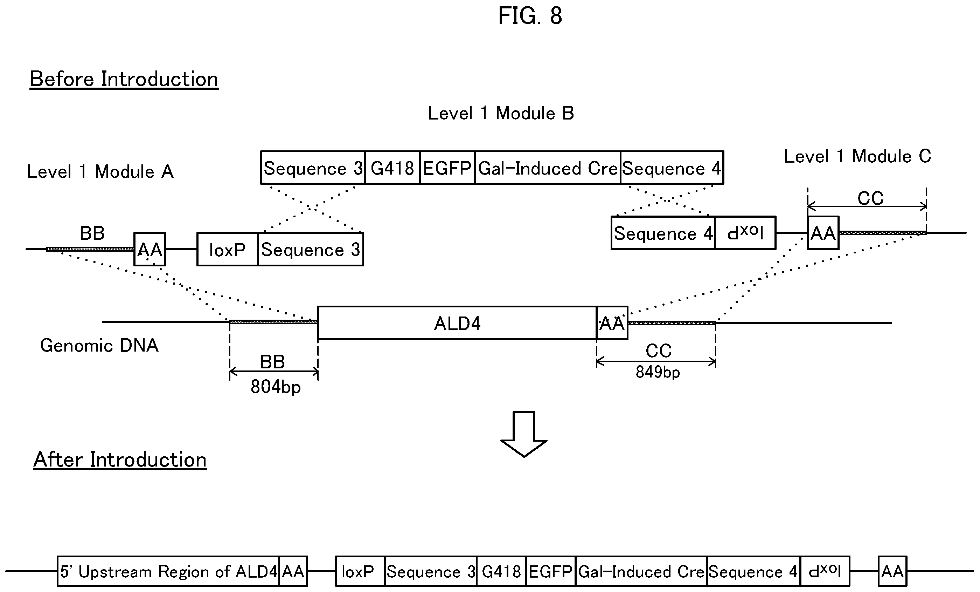

Fig. 8 is an explanatory diagram illustrating the state of introducing the sequence structure shown in Fig. 1 into an ALD4 gene locus of the Saccharomyces genome. As in the case of Fig. 6, in this illustrated example, loxP sequences are used as the target sequences 12a and 12b, and the homologous sequence 14 is shown as a homologous sequence AA. As shown in Fig. 8, the homologous sequence AA was a sequence in a range of 50 bp from a 3'-terminal side of an ECM38 protein coding region (CDS) in the Saccharomyces genomic DNA as an object to be introduced. A sequence having the length of 50 bp was accordingly used as the homologous sequence AA (as shown in Fig. 9, Fig. 10 and Fig. 13 described later).

As shown in Fig. 6 to Fig. 8, all the vectors under test for Saccharomyces cerevisiae have been designed, such that mixing and genetic transformation of a three-fragment set causes a recombination between homologous areas (one of the sequences 1 to 4) of the respective fragments and eventually obtains one fragment, which is to be introduced into the Saccharomyces genome. In Fig. 6 to Fig. 8, the respective fragments of the three-fragment set to obtain one fragment, which is to be introduced into the Saccharomyces genome, are shown as a level 1 module A, a level 1 module B and a level 1 module C in a sequence from a 5'-side after binding.

Fig. 9 is an explanatory diagram showing the configurations of respective level 1 modules used to be introduced into the Saccharomyces genome shown in Fig. 6 to Fig. 8. Fig. 9 collectively shows the type of the destination vector for level 1 used (shown in Fig. 4), the type of a level 0 module, and the configuration of a level 0 module insert with regard to each of the level 1 modules. Fig. 9 also shows level 1 modules used for evaluation of the efficiency of homologous recombination in Escherichia coli described later.

The following describes a procedure of producing the respective level 1 modules shown in Fig. 9. A sequence B (shown in Fig. 6), which was a sequence in part of an ECM38 protein coding region (CDS) used for homologous recombination with genome and which was a sequence of 1080 bp from the top of ORF of ECM38, and a sequence A having the length of 50 bp as described above were amplified by PCR using the genome of an S. cerevisiae BY4742 strain, the genome of an Escherichia coli K12 strain or a synthetic DNA as the template. The DNA sequences were sequentially bound to one another by using In-Fusion HD Cloning Kit, and a resulting target DNA fragment was subcloned on a vector (shown in Fig. 3) corresponding to the destination vector for level 0 of the Golden Gate method. The obtained module was designated as a level 0 module V1P1042. Level 0 modules V1P1087, V1P1245, V1P1246 and CIP1260 were also obtained as level 0 modules similar to the level 0 module V1P1042 by respectively adding four different types of sequences (50 bp, 100 bp, 200 bp and 1000 bp) serving as a spacer sequence (first spacer sequence Spu shown in Fig. 1) between the loxP sequence and the sequence A to a 3' downstream region of the insert of V1P1042. Level 0 modules V1P1265 and V1P1263 were also obtained as level 0 modules similar to the level 0 module V1P1246 by changing the length of the sequence A to 20 bp and to 30 bp, and a level 0 module V1P1248 was obtained by excising the sequence A from the insert of V1P1042 (as shown in Fig. 9). The spacer sequence (the first spacer sequence Spu) used was a partial sequence of the CDS of the araB gene in the Escherichia coli K12 strain. These level 0 modules are used to obtain the level 1 module A shown in Fig. 6 and Fig. 7.

Similarly, a sequence BB (shown in Fig. 8) as a DNA fragment including a homologous recombination region in a range of 804 bp from a 5'-terminal side of the CDS of the ALD4 gene for homologous recombination with the genome and a sequence AA having the length of 50 bp as described above were subcloned, and the obtained module was designated as a level 0 module V1P1262. A level 0 module V1P1274 similar to the level 0 module V1P1262 was obtained as a vector by adding a sequence (200 bp) serving as a spacer sequence (first spacer sequence Spu shown in Fig. 1) between the loxP sequence and the sequence AA to a 3' downstream region of the insert of V1P1262. These level 0 modules are used to obtain the level 1 module A shown in Fig. 8.

Fused DNA fragments of G418-resistant gene (G418 marker), EGFP gene as altered GFP gene (Gene, 173, 33-38 (1996)), and Cre gene bound to a promoter of GAL10 gene induced by galactose were subcloned on the destination vector for level 0 (shown in Fig. 3), and the obtained module was designated as a level 0 module V1P256 (as shown in Fig. 9). This is used to obtain the level 1 module B shown in Figs. 6 to 8.

A DNA fragment including a homologous recombination region of 841 bp from 301 bp of a 3'-terminal side of the CDS of the ECM38 gene in the Saccharomyces genomic DNA as the object to be introduced (sequence C shown in Fig. 6 and Fig. 7) was subcloned on the destination vector for level 0 (shown in Fig. 3), and the obtained module was designated as a level 0 module V1P70 (as shown in Fig. 9). Furthermore, level 0 modules V1P1261 and V1P1249 (shown in Fig. 9) were obtained as vectors by respectively adding spacer sequences (200 bp, 50 bp) (second spacer sequence Spd shown in Fig. 1) between the loxP sequence and the homologous recombination sequence A to a 5' upstream region of the insert of the level 0 module V1P70. The spacer sequence (the second spacer sequence Spd) used was a partial sequence of the CDS of the araA gene in the Escherichia coli K12 strain. These level 0 modules are used to obtain the level 1 module C shown in Fig. 6 and Fig. 7.

Similarly, a DNA fragment including a homologous recombination region of 849 bp from a 3'-terminal side of the CDS of the ALD4 gene in the Saccharomyces genomic DNA as the object to be introduced (sequence CC shown in Fig. 8 and Fig. 7) was subcloned on the destination vector for level 0 (shown in Fig. 3), and the obtained module was designated as a level 0 module V1P73 (as shown in Fig. 9). This level 0 module was used to obtain the level 1 module C shown in Fig. 8.

Level 1 modules were produced by the Golden Gate reaction (using BsaI as the restriction enzyme) of the vectors of the respective 0 modules described above with the corresponding destination vectors for level 1 shown in Fig. 9. Each combination of three different level 1 modules obtained was introduced as a V3P vector set into the Saccharomyces genome as shown in Figs. 6 to 8.

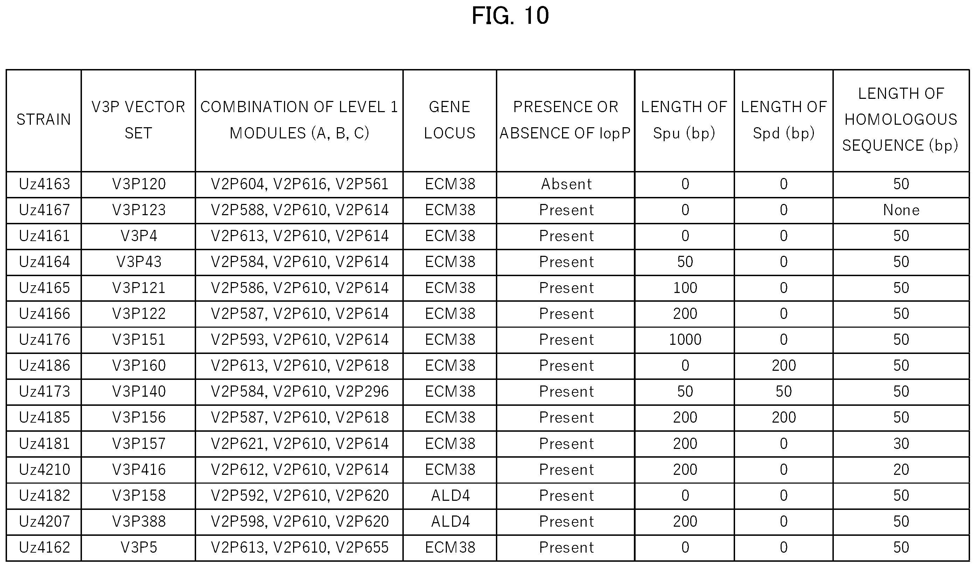

Fig. 10 is an explanatory diagram showing the configurations of V3P vector sets introduced into the Saccharomyces genome. Fig. 10 shows the combination of three different level 1 modules configuring each of the V3P vector sets, the name of the strain of Saccharomyces cerevisiae with each of the V3P vector sets introduced therein, the gene locus with the sequence structure of Fig. 1 introduced therein, the presence or absence of the loxP sequence in each of the V3P vector sets, the length of the first spacer sequence Spu, the length of the second spacer sequence Spd, and the length of the homologous sequence (A or AA). As shown in Figs. 6 to 8, each of the V3P vector sets includes, as the insert thereof, a homologous recombination sequence (the sequence B or the sequence BB) that allows for homologous recombination with a 5' upstream region of an introducing location in the Saccharomyces genomic DNA, a homologous recombination sequence (the sequence C or the sequence CC) that allows for homologous recombination with a 3' upstream region of the introducing location in the Saccharomyces genomic DNA, or a sequence selected from the sequences 1 to 4 that allow for homologous recombination between the respective inserts. Genetic transformation of the three different inserts of the V3P plasmid set as shown in Fig. 10 causes homologous recombination between the respective sequences and homologous recombination with the Saccharomyces genomic DNA. This causes all the fragments to be eventually bound to one another and to be introduced into the Saccharomyces genome in a sequence shown as the "combination of level 1 modules" in Fig. 10.

### <Introduction of Vector into Saccharomyces Genome>

An insert from each plasmid of each of the vector sets produced was amplified by PCR, and genetic transformation of the Saccharomyces cerevisiae BY4742 strain was performed by using the respective amplified fragments. The transformed Saccharomyces cerevisiae BY4742 strain was applied on a YPD agar medium including G418, and grown colonies were purified. A strain having homologous recombination as expected was selected by PCR. The genetic transformation was performed according to a method of Akada et al. (Biotechniques 28, 854 (2000)). Fig. 10 shows a produced transformant of Saccharomyces cerevisiae.

### <Induction of Homologous Recombination into Saccharomyces cerevisiae and Measurement of Efficiency of Homologous Recombination>

The obtained strain was cultured at a concentration of 100 or 1000 cells/ plate in a YPGA agar medium (10 g/L of Saccharomyces cerevisiae extract, 20 g/L of peptone, 20 g/L of galactose), and expression of the Cre gene was induced. Homologous recombination occurring accompanied with the expression of the Cr gene is thought to cause omission of a marker gene, an EGFP gene and a Cre gene placed between homologous sequences (the sequences A or the sequences AA) as a set and thereby quench the fluorescence of the EGFP. Among the grown colonies, colonies having any part of fluorescence quenching were counted as candidates of colonies subjected to homologous recombination. The extinction factor of the colonies to all the colonies as the denominator was calculated. As a control, with regard to a Uz4161 strain, the efficiency was measured under YPD medium conditions with no induction of the expression of the Cre gene. Furthermore, with regard to part of strains, PCR was performed for the colonies with quenching by using primers set to be placed on respective sides of a genome introducing site. It was then evaluated whether homologous recombination was performed accurately in the amplified DNA size level.

### <Production of Vectors for Escherichia coli>

All the vectors used for the test were produced by the two-step Golden Gate method and were evaluated as the cyclic plasmid with respect to the efficiency of homologous recombination with Escherichia coli. The sequence for homologous recombination used was the sequence A for Saccharomyces cerevisiae, and the vectors used were shared by the vectors for Saccharomyces cerevisiae (shown in Fig. 9). Some modules were additionally produced.

In order to obtain the vectors for homologous recombination, a gene sequence (SpR marker) including spectinomycin-resistant gene and mRFP1.1 gene (Nat Biotechnol 22, 1567 (2004)) were amplified by PCR using the genomic DNA of the Escherichia coli K12 strain and a synthetic DNA as the templates and were subcloned on the destination vector for level 0 (shown in Fig. 3). The obtained module was designated as a level 0 module V1P415 (shown in Fig. 9).

Level 1 modules were then produced by the Golden Gate reaction (using BsaI as the restriction enzyme) of the vectors of the respective level 0 modules and the destination vectors for level 1 shown in Fig. 9. The respective level 1 modules were completed as level 2 vectors by the second Golden Gate reaction (using Eps3I as the restriction enzyme) with the destination vector for level 2 P1301 (shown in Fig. 5).

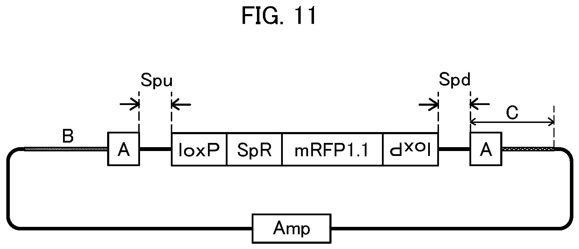

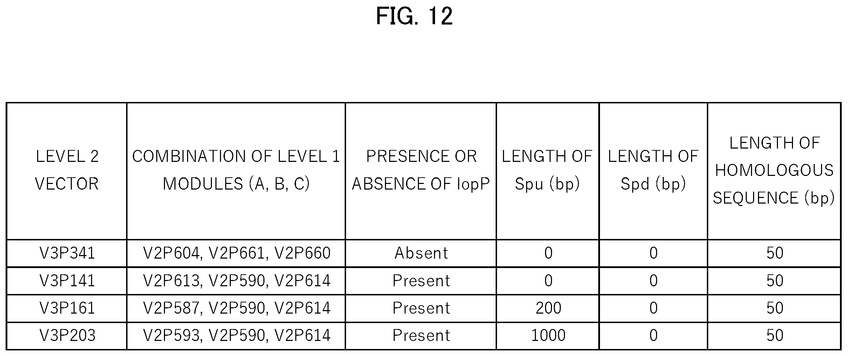

Fig. 11 is an explanatory diagram illustrating a typical configuration of a level 2 vector for Escherichia coli, which was produced as described above for the purpose of evaluation of the efficiency of homologous recombination. Fig. 12 is an explanatory diagram showing the details of respective level 2 vectors introduced into Escherichia coli.

Separately from the level 2 vectors described above, a vector for expression of Cre gene was produced. Fused DNA fragments of tetR gene required for a Tet on system induced by tetracycline and Cre gene bound to a tetA promoter were amplified by PCR using the genomic DNA of the Escherichia coli K12 strain and the synthetic DNA as the templates and were subcloned on the destination vector for level 0 (shown in Fig. 3). The obtained module was designated as a level 0 module V1P408. A level 1 module V2P591 was then produced (as shown in Fig. 9) by the Golden Gate reaction (using BsaI as the restriction enzyme) of this level 0 module V1P408 and the destination vector for level 1 V1P402 (shown in Fig. 4).

### <Production of Strain for Evaluation of Homologous Recombination in Escherichia coli>

Each of the level 2 vectors shown in Fig. 12 and the vector V2P591 for expression of Cre gene were simultaneously transformed in an Escherichia coli DH5α strain or an Escherichia coli BL21 (DE3) strain, and obtained transformants were used for an experiment of homologous recombination. Strains obtained by genetic transformation of only the level 2 vectors were also evaluated as controls.

### <Induction of Homologous Recombination in Escherichia coli and Measurement of Efficiency of Homologous Recombination>

The homologous recombination is expected to cause quenching of fluorescence of mRFP1.1. In a medium where Escherichia coli with genetic transformation of two vectors was grown, most of the transformed colonies were quenched. This was expected to suggest the occurrence of homologous recombination with weak expression of the Cre gene. Colonies having any portions of fluorescence quenching out of all the grown colonies were counted as candidate colonies of homologous recombination. An extinction factor of such colonies to all the colonies as the denominator was examined. Part of the colonies with quenching was isolated and cultured in an LB medium with addition of anhydrotetracycline to induce the expression of the Cre gene. PCR was then performed by using primers set to be placed on respective sides of each homologous recombination region, and the efficiency of homologous recombination was calculated from the band size.

### <Results of Evaluation>

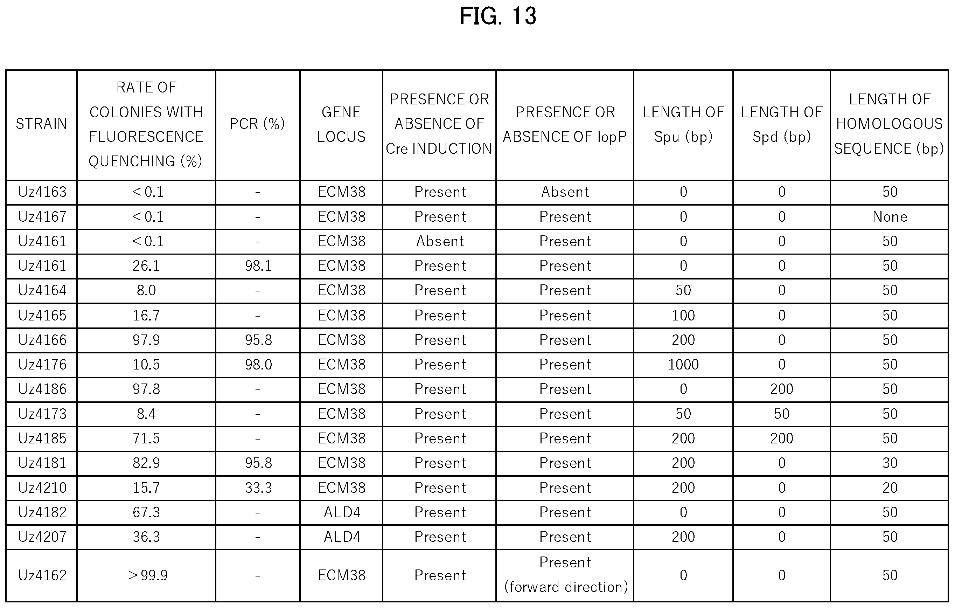

Fig. 13 is an explanatory diagram showing the results of an experiment of homologous recombination in Saccharomyces cerevisiae. As shown in Fig. 13, in the case of Saccharomyces cerevisiae, no colonies having quenching were observed under the conditions of controls satisfying any one of: (i) the strain had no homologous sequences at locations on the outside of a pair of loxP sequences (Uz4167 strain); (ii) the strain had no loxP sequences (Uz4163 strain); and (iii) the strain had all the constitutional elements but was under the condition of inducing no expression of the Cre gene (Uz4161 strain). These results suggest no occurrence of homologous recombination in the controls. Compared with the controls, on the other hand, the fluorescence quenching was observed at the efficiency of at least hundredfold or higher in any of the strains that had two loxP sequences of different directionalities, irrespective of the presence or the absence of the spacer sequence (either the first spacer sequence Spu or the second spacer sequence Spd), that had a pair of homologous sequences on the outside of the loxP sequences, and that were under the condition of inducing expression of the Cre gene. In some cases, fluorescence quenching was observed in most of the cells under certain conditions. According to the results of PCR, when the region of homologous recombination (the length of the homologous sequences) is 30 bp or greater, it is thought that correct homologous recombination occurs in 90% or more of the colonies having the recombination. When the length of the homologous sequences is 20 bp, however, the ratio of correct homologous recombination is lowered to approximately 33%. Based on these results, the length of the homologous sequences is preferably 25 bp or greater. The survival rate of the cells (counted from the number of the colonies) grown in the Cre gene-inducing medium is 10 to 100%. It is thus considered that homologous recombination occurs in the grown cells at the probability of 10⁻⁰ to 10⁻² under any conditions. The results shown in Fig. 13 accordingly suggest the occurrence of homologous recombination at an extremely higher frequency including the case where the length of the homologous sequences is 20 bp, compared with the value of 10⁻⁶ known as the efficiency of genome homologous recombination in Saccharomyces cerevisiae by introduction of a foreign gene (as shown in Non-Patent Literature 1).

In the case of the Uz4161 strain, sequencing was performed for 5-clone estimated to have correct recombination in the DNA size level, and the occurrence of correct homologous recombination was confirmed. It was also confirmed that simple subculture of the cells having fluorescence quenching without isolation caused most of the cells to quench the fluorescence and to be changed a cell population having homologous recombination, even under conditions of poor recombination efficiency. In the case where the pair of target sequences (loxP sequences) are placed in an identical direction (forward direction) (in the case of the Uz4162 strain), the site-specific recombination reaction proceeded in the pair of loxP sequences placed in the forward direction and increased the rate of the colonies having fluorescence quenching to an extremely high value of not lower than 99.9%. This may be attributable to that the site-specific recombination reaction in the pair of loxP sequences placed in the forward direction stabilizes the DNA molecule (Saccharomyces genome) in the state that the specific DNA sequence 15 is excised between the pair of loxP sequences.

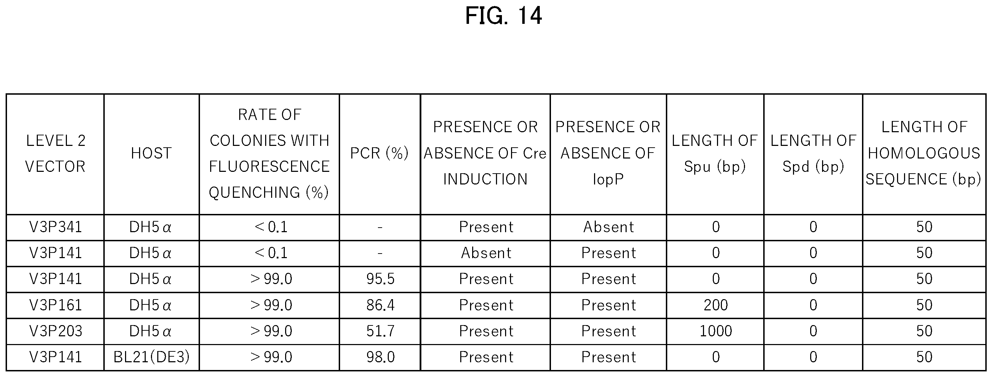

Fig. 14 is an explanatory diagram showing the results of an experiment of homologous recombination in Escherichia coli. As shown in Fig. 14, in the case of Escherichia coli, no colonies having fluorescence quenching were observed under the conditions of the controls as in the case of Saccharomyces cerevisiae. The fluorescence quenching was observed, on the other hand, in almost all the colonies in any of the strains that had two loxP sequences of different directionalities, irrespective of the presence or the absence of the spacer sequence (the first spacer sequence Spu), that had a pair of homologous sequences on the outside of the loxP sequences, and that were under the condition of inducing expression of the Cre gene. A relatively long interval between the loxP sequence and the homologous sequence A (i.e., the length of the first spacer sequence Spu), however, tended to slightly decrease the probability of correct homologous recombination. In Escherichia coli, it has been conventionally known that in the case of not using a Lambda phage-derived homologous recombination enzyme for genome homologous recombination by genetic transformation, relatively short homologous sequences of about 50 bp do not cause homologous recombination. It has also been conventionally known that even in the case of using the Lambda phage-derived homologous recombination enzyme, the efficiency of homologous recombination is about 10⁻³ in the homologous sequences having the length of 50 bp (Front. Mirobiol., 11 September 2020, https://doi.org/10.3389/fmicb.2020.548410). According to the results shown in Fig. 14, on the other hand, the grown cells have homologous recombination at the probability of 10⁻⁰ to 10⁻¹ in the homologous sequences of 50 bp. This indicates homologous recombination of the high frequency that is not assumable by the conventional techniques.

As described above, the homologous recombination method using the sequence structure shown in Fig. 1 similarly achieved the high efficiency of homologous recombination not only in the case of Saccharomyces cerevisiae but in the case of organism species unlikely to have homologous recombination as in the case of Escherichia coli using the homologous sequences having the length of about 50 bp. Accordingly, the homologous recombination method using the sequence structure shown in Fig. 1 is considered to enhance the efficiency of homologous recombination as a universal reaction in a wider range of organism species regardless of the intrinsic efficiency of homologous recombination of each host, irrespective of whether the object of recombination is a prokaryote or a eukaryote.

The present disclosure is not limited to the above embodiments described above but may be implemented in a variety of configurations without departing from the subject matter or the scope of the present disclosure. For example, the technical features of the embodiments corresponding to the technical features of the respective aspects described in Summary may be appropriately changed, altered, replaced or combined, in order to solve part or the entirety of the problems described above or in order to achieve part or the entirety of the advantageous effects described above. The technical features may be appropriately omitted unless the technical features are mentioned as essential in the description hereof.

The present disclosure may be implemented by aspects described below.
[Aspect 1] A homologous recombination method of excising a specific DNA sequence from a DNA molecule, comprising:
   providing the DNA molecule that has a pair of target sequences as a target of a site-specific recombination enzyme are placed in different directions from each other across the specific DNA sequence and that a pair of homologous sequences as DNA sequences sharing homology with each other are placed on further outside of the pair of target sequences placed across the specific DNA sequence; and
   making the site-specific recombination enzyme act on the DNA molecule and thereby causing homologous recombination between the pair of homologous sequences, so as to excise the specific DNA sequence along with the pair of target sequences from the DNA molecule.
[Aspect 2] The homologous recombination method according to aspect 1,
   wherein the site-specific recombination enzyme is an enzyme belonging to an integrase family and causes a reversible reaction to proceed as a recombination reaction of the target sequences.
[Aspect 3] The homologous recombination method according to aspect 2,
   wherein the site-specific recombination enzyme is an enzyme belonging to a bidirectional tyrosine recombinase subfamily.
[Aspect 4] The homologous recombination method according to aspect 3,
   wherein the site-specific recombination enzyme is an enzyme selected among Cre recombinase, FLP recombinase, R recombinase and Dre recombinase.
[Aspect 5] The homologous recombination method according to any one of aspects 1 to 4, further comprising:
   using Saccharomyces cerevisiae or Escherichia coli as a target cell of homologous recombination, wherein
   a distance between the target sequence and the homologous sequence placed adjacent to each other either on an upstream side or on a downstream side of the specific DNA sequence is 1000 bp or less.
[Aspect 6] The homologous recombination method according to any one of aspects 1 to 5,
   wherein the homologous sequence used is a sequence having a length of 60 bp or less.
[Aspect 7] The homologous recombination method according to any one of aspects 1 to 6,
   wherein the homologous sequence used is a sequence having a length of 25 bp or greater.

### Reference Signs List

- 10: DNA molecule
- 12a, 12b: target sequences
- 14: homologous sequence
- 15: specific DNA sequence

## Claims

1. A homologous recombination method of excising a specific DNA sequence from a DNA molecule, comprising:
providing the DNA molecule that has a pair of target sequences as a target of a site-specific recombination enzyme are placed in different directions from each other across the specific DNA sequence and that a pair of homologous sequences as DNA sequences sharing homology with each other are placed on further outside of the pair of target sequences placed across the specific DNA sequence; and
making the site-specific recombination enzyme act on the DNA molecule and thereby causing homologous recombination between the pair of homologous sequences, so as to excise the specific DNA sequence along with the pair of target sequences from the DNA molecule.

2. The homologous recombination method according to claim 1, wherein the site-specific recombination enzyme is an enzyme belonging to an integrase family and causes a reversible reaction to proceed as a recombination reaction of the target sequences.

3. The homologous recombination method according to claim 2, wherein the site-specific recombination enzyme is an enzyme belonging to a bidirectional tyrosine recombinase subfamily.

4. The homologous recombination method according to claim 3, wherein the site-specific recombination enzyme is an enzyme selected among Cre recombinase, FLP recombinase, R recombinase and Dre recombinase.

5. The homologous recombination method according to any one of claims 1 to 4, further comprising:
using Saccharomyces cerevisiae or Escherichia coli as a target cell of homologous recombination, wherein
a distance between the target sequence and the homologous sequence placed adjacent to each other either on an upstream side or on a downstream side of the specific DNA sequence is 1000 bp or less.

6. The homologous recombination method according to any one of claims 1 to 5,
wherein the homologous sequence used is a sequence having a length of 60 bp or less.

7. The homologous recombination method according to any one of claims 1 to 6,
wherein the homologous sequence used is a sequence having a length of 25 bp or greater.
